# EUROPEAN PATENT APPLICATION

(11) **EP 1 970 095 A1**
(43) Date of publication of application: **17.09.2008**
(21) Application number: 06843190.7
(22) Date of filing: 25.12.2006
(51) Int. Cl.: A61N 1/30

(54) **IONTOPHORESIS DEVICE AND METHOD FOR PRODUCING SAME**

(30) Priority: 27.12.2005 JP 2005374155
(71) Applicant: Hisamitsu Pharmaceutical Co., Inc., Tosu-shi, Saga 841-0017 (JP); Kyodo Printing Co., Ltd., Tokyo 112-8501 (JP)
(72) Inventor: TOKUMOTO, Seiji, Ibaraki 305-0856 (JP); ADACHI, Hirotoshi, Ibaraki 305-0856 (JP); FUCHITA, Yasushi, Tokyo 112-8501 (JP); TAKAHASHI, Saori, Tokyo 112-8501 (JP); OGAWA, Tatsuya, Tokyo 112-8501 (JP)
(74) Representative: Shindler, Nigel
(86) International application number: PCT/JP2006/325787
(87) International publication number: WO 2007/077796

(57) **Abstract**

To provide an easily producible mass-production type iontophoresis device having a structure that a dissolution liquid-storing container is integrated with an iontophoresis electrode, a dissolution liquid and a drug can be mixed by simple operations, and it is free from a risk of leakage of electricity. The device comprises an electrode film having an electrode layer (2) formed on a base (1), a drug-loaded member (3) arranged on the electrode-layer-forming side of the electrode film, and a dissolution liquid-storing container (5) arranged on the non-electrode-layer-forming side of the electrode film, wherein the electrode film is provided witH a dissolution liquid passing hole (9), a resin lid member (7) is bonded to the electrode film via a thermoplastic resin layer (10) or an adhesive layer to cover the dissolution liquid passing hole, and a flange (5a) of the dissolution liquid-storing container (5) is bonded to the electrode film via the resin lid member (7).

## Description

### TECHNICAL FIELD

The present invention relates to an iontophoresis device used in the medical field of treatment or diagnosis.

### BACKGROUND ART

The iontophoresis is a type of method for enhancement of physical absorption of a drug and administers the drug through the skin or the mucosa by applying voltage to the skin or the mucosa and electrically causing migration of the drug.

In a case where a drug which is particularly poor in stability to water is used in the iontophoresis device, it is necessary to separate the drug and a dissolution liquid from each other at the time of storage in order to prevent the drug from being deteriorated during its storage and to mix the drug and the dissolution liquid immediately before the use for treatment. For that, it is convenient to have a structure that a dissolution liquid storing container is integrated with the iontophoresis electrode itself which includes an electrode layer to be applied to the skin or the mucosa, and the dissolution liquid and the drug can be mixed by a simple operation.

For example, Patent Literature 1 proposes a structure that a capsule in which an electrolyte solution is encapsulated is fitted to a plaster structure for iontophoresis which is composed of an electrode layer and a drug-containing layer via an aluminum foil.

### [Patent Literature 1]

Japanese Patent Publication No. Hei 5-84180

### SUMMARY OF THE INVENTION

### [Problems to be solved by the Invention]

The structure proposed by Patent Literature 1 can mix an electrolyte and a drug by breaking the aluminum foil but has disadvantages that there is a possibility of causing a leakage of electricity from an aluminum exposed portion when current is applied resulting in a high risk of giving an electrical shock to a human body and it lacks safety.

A disposable iontophoresis device is questioned for its mass productivity, and its structure and production method that mass production can be made easily are needed. But, the structure proposed in Patent Literature 1 has problems that it lacks productivity, mass production is not easy and the cost increases.

The present invention has been made in view of the above circumstances and has an object to overcome the above problems and to provide a mass production type iontophoresis device that a dissolution liquid storing container is integrated with an iontophoresis electrode itself, it has a structure capable of mixing a dissolution liquid and a drug by a simple operation, it is free from a possibility of causing a leakage of electricity and easy production is made possible, and a method for producing it.

### [Means for solving the Problems]

The present invention configured to remedy the above-described problems has the following structures.

(1) An iontophoresis device, comprising at least an electrode film having an electrode layer formed on a base, a drug-loaded member arranged on the electrode-layer-forming side of the electrode film, and a dissolution liquid storing container arranged on the non-electrode-layer-forming side of the electrode film, wherein the electrode film is provided with a dissolution liquid passing hole, a resin lid member is bonded to the electrode film via a thermoplastic resin layer or an adhesive layer to cover the dissolution liquid passing hole, and a flange of the dissolution liquid storing container is bonded to the electrode film via the resin lid member.
(2) The iontophoresis device according to (1), wherein the surface of the resin lid member positioned at the dissolution liquid passing hole does not have the thermoplastic resin layer or the adhesive layer.
(3) A method for producing an iontophoresis device which comprises at least an electrode film having an electrode layer formed on a base, a drug-loaded member arranged on the electrode-layer-forming side of the electrode film, and a dissolution liquid storing container arranged on the non-electrode-layer-forming side of the electrode film, comprising:
   laminating a thermoplastic resin layer on the entire surface on the side of the electrode film to which the dissolution liquid storing container is bonded,
   forming a dissolution liquid passing hole in the electrode film,
   bonding a resin lid member to the thermoplastic resin layer by a heat lamination method, and
   bonding the dissolution liquid storing container to the resin lid member.
(4) A method for producing an iontophoresis device which comprises at least an electrode film having an electrode layer formed on a base, a drug-loaded member arranged on the electrode-layer-forming side of the electrode film, and a dissolution liquid storing container arranged on the non-electrode-layer-forming side of the electrode film, comprising:
   forming a dissolution liquid passing hole in the electrode film,
   laminating a thermoplastic resin layer on a resin lid member,
   bonding the resin lid member to the electrode film via the thermoplastic resin layer to cover the dissolution liquid passing hole by a heat lamination method, and
   bonding the dissolution liquid storing container to the resin lid member.
(5) A method for producing an iontophoresis device which comprises at least an electrode film having an electrode layer formed on a base, a drug-loaded member arranged on the electrode-layer-forming side of the electrode film, and a dissolution liquid storing container arranged on the non-electrode-layer-forming side of the electrode film, comprising:
   forming an adhesive layer on only a part of the electrode film to which the dissolution liquid storing container is bonded,
   forming a dissolution liquid passing hole in a region of the inner side of the electrode film where the adhesive layer is formed,
   bonding a resin lid member to a flange of the dissolution liquid storing container, and
   bonding the resin lid member bonded to the dissolution liquid storing container to the electrode film via the adhesive layer to cover the dissolution liquid passing hole.
(6) A method for producing an iontophoresis device which comprises at least an electrode film having an electrode layer formed on a base, a drug-loaded member arranged on the electrode-layer-forming side of the electrode film, and a dissolution liquid storing container arranged on the non-electrode-layer-forming side of the electrode film, comprising:
   forming a dissolution liquid passing hole in the electrode film,
   bonding the non-adhesive-layer-forming side of a resin lid member which has an adhesive layer formed on its one surface to a flange of the dissolution liquid storing container, and
   bonding the resin lid member which is bonded to the dissolution liquid storing container to the electrode film via the adhesive layer to cover the dissolution liquid passing hole.

### EFFECTS OF THE INVENTION

The iontophoresis device of the present invention has a structure capable of mixing the dissolution liquid and the drug by a simple operation and uses the insulating resin lid member as the lid member of the dissolution liquid storing container, so that there is no possibility of a leakage of electricity when applying current and its reliability and safety are excellent.

According to the method for producing the iontophoresis device of the present invention, the electrode film or the electrode film with the resin lid member can be mass-produced by using a material such as a rolled sheet or the like, and the iontophoresis device excelling in reliability and safety can be mass-produced easily.

### BEST MODE FOR CARRYING OUT THE INVENTION

Embodiments of the present invention are described below with reference to the drawings.

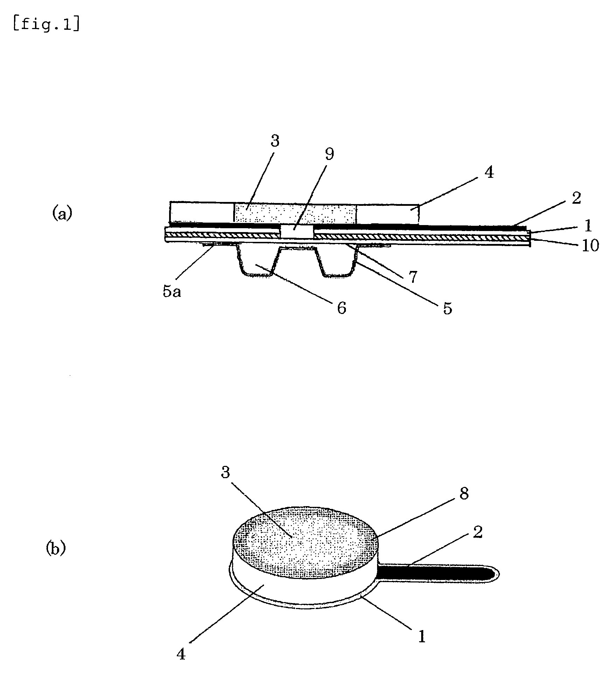

Fig. 1 is a diagram showing an embodiment of the iontophoresis device according to the present invention, where (a) is a sectional view, and (b) is a perspective view. The iontophoresis device of this embodiment is mainly comprised of a base 1 formed of a polyethylene terephthalate film (PET) or the like, an electrode layer 2 formed on the base 1, a drug-loaded member 3 formed on the electrode layer 2, an expanded sheet 4 arranged along the circumference of the drug-loaded member 3, a dissolution liquid storing container (dissolution liquid-storing blister container) 5 in which a dissolution liquid 6 is charged, and a lid member 7 which functions as a lid member of the dissolution liquid storing container 5.

### (Electrode film)

As the base 1 of the electrode film, there are, for example, plastic films of polyethylene terephthalate (PET), polyimide, polyamide, polypropylene and the like, and the polyethylene terephthalate is particularly suitably used because it is excellent in insulation properties, heat resistance, machinability and the like. And, such films may be used as a single film or a composite film.

The electrode layer 2 is composed of a substantially circular part which becomes an electrode discharge portion and an extended part which becomes an electrode terminal portion. As a material for the electrode layer 2, for example, materials based on metal and nonmetal conductive materials such as silver, silver chloride, carbon, titanium, platinum, gold, aluminum, iron, nickel and a mixture of them can be used. A conductive paste based on such a material may also be used. By using such a conductive paste, the electrode layer can be formed by screen printing suitable for mass production.

For the electrode film having the electrode layer 2 formed on the base 1, a hole which becomes a dissolution liquid passing portion 9 to move the dissolution liquid 6 in the dissolution liquid storing container 5 to the drug-loaded member 3 at the time of use is formed by punching fabrication.

### (Drug-loaded member)

The material for the drug-loaded member 3 is not limited to a particular one if it is a hydrophilicity based one, can absorb and hold a drug solution, and may be a cellulose fiber, a rayon fiber, a nylon fiber, a polyurethane foam, a polycarbonate foam, a polyvinyl alcohol foam, a polyester foam, a polystyrene foam, a polyester nonwoven fabric, a polyester nonwoven fabric, cotton or a composite of them.

### (Expanded sheet)

The expanded sheet 4 has a function to prevent the drug solution from externally leaking out of the device when the drug-loaded member 3 is impregnated with the dissolution liquid. Therefore, it is required to be securely adhered to the electrode film. And, since the device is used in a state attached to the skin, it is desired that the expanded sheet is a flexible soft foam having as a substrate various types of polymers such as polyurethane, polyethylene, polyvinyl chloride, polychloroprene, acrylic resin and polystyrene foams. And, since it is attached to the skin when used, it is desirable to have an adhesive 8 such as rubber-based, acrylic-based, silicone-based, polyvinyl-based, polyester-based, polyurethane-based or the like coated onto one side.

### (Sealing layer)

The drug-loaded member 3 and the expanded sheet 4 are bonded to the electrode film with a sealing layer (not shown), which is formed of an adhesive material or a heat-sealable material, provided in a prescribed region such as a peripheral portion of the electrode layer of the electrode film. The sealing layer is preferably heat sealable in view of an easy production process. Heat sealable materials are polydiene, polyacryl, polymethacryl, acrylamide, polyvinyl alcohol, polyethylene, polyvinyl ester, polystyrene, polycarbonate, polyester, polyurethane, polysiloxane, polyamide, polyacetal and polyacrylonitrile. Preferably, they are polydiene, polyacryl, polymethacryl, polyethylene, polyvinyl ester, polystyrene, polyester and polysiloxane. More preferably, they are polydiene, polyacryl, polymethacryl, polyester and polysiloxane but not limited to them. Adhesive materials are those having acryl or silicone as the main component but not limited to them.

### (Dissolution liquid-storing blister container)

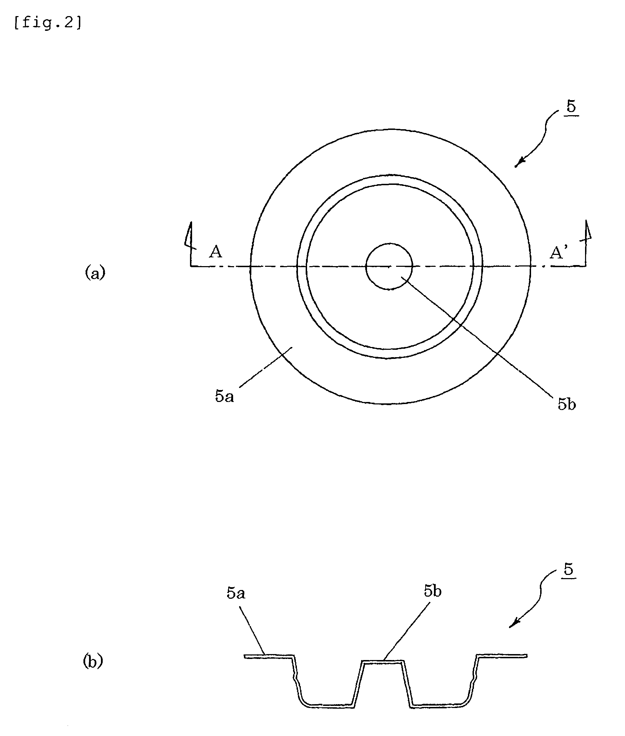

Fig. 2 is a diagram showing the dissolution liquid-storing blister container 5 of this embodiment, where (a) is a top view, and (b) is an A-A' sectional view of (a). As shown in the drawing, the dissolution liquid-storing blister container 5 has a flange portion 5a at the top of the outer circumference and a projected portion 5b at the center in the container.

For the dissolution liquid-storing blister container 5, it is desired to use a material having a high steam barrier property to prevent the dissolution liquid 6 from evaporating and decreasing its amount when the dissolution liquid 6 is charged and stored in the dissolution liquid-storing blister container 5. Such a material includes polyvinyl chloride, polyvinylidene chloride, polyethylene, polypropylene, polystyrene, polyamide, polymethylmethacrylate, polycarbonate, polyester, polyvinyl alcohol, polyvinyl acetate, thermoplastic elastomer, cyclic olefin polymer, cyclic olefin copolymer,a copolymer of ethylene with an organic carboxylic acid derivative having an ethylenically unsaturated bond, such as ethylene-methacrylate copolymer, ethylene-vinyl acetate copolymer, ethylene-acrylic acid copolymer and ethylene-vinyl acetate-methyl methacrylate copolymer, trifluoroethylene chloride resin, and the like. The dissolution liquid-storing blister container can be obtained by vacuum molding, compressed air molding or vacuum compressed air molding of a single layer or a sheet formed of laminated multilayers.

### (Lid member)

The lid member 7 is required to be excellent in a steam barrier property and a break-through characteristic such that permeation of moisture to the drug-loaded member 3 is prevented during storage and it is broken easily to mix the electrolyte 6 of the blister container 5 and the drug of the drug-loaded member 3 when used. And, since the iontophoresis system essentially needs application of current when it is used, if a conductive material such as an aluminum foil is used for the lid member, there is a possibility of a leakage of electricity. Therefore, the present invention uses as the lid member 7 a resin lid member of an insulating material. And, a half-cut line may be formed in the resin lid member to facilitate its break through.

The materials for the resin lid member 7 include for example polyester, polystyrene, polyolefin such as polypropylene, an ethylene-propylene copolymer, polyethylene and the like, and it is suitably a uniaxially-stretched sheet or a sheet with an inorganic filler of calcium carbonate or the like blended into the above material.

It is desirable that the present invention bonds the electrode film and the lid member via a thermoplastic resin layer (or an adhesive layer) 10.

Usable for the thermoplastic resin includes a sealant resin such as polypropylene based resins and polyethylene based resins such as low-density polyethylene, medium-density polyethylene, high-density polyethylene, linear low-density polyethylene, ethylene-vinyl acetate copolymer, ethylene-acrylic acid copolymer, ethylene-methacrylic acid copolymer, ethylene-ethylacrylate copolymer, ethylene-methyl-methacrylate copolymer, ethylene-methacrylate copolymer, ionomer, and a derivative or mixture of them.

In a case where the electrode film and the lid member are bonded via the thermoplastic resin layer, the thermoplastic resin is sandwiched between them, and they are laminated. As a laminating method, various types of fabrication methods such as an explosion lamination method, a dry lamination method, a heat lamination method and the like can be used. The thermoplastic resin is laminated by extruding by a T-die according to the explosion lamination method, and laminated by forming a film by inflation or the T-die method according to the dry and heat lamination method. In a case where the electrode film in which the dissolution liquid passing hole is formed is laminated, the heat lamination method is used because the explosion lamination method and the dry lamination method cannot be applied.

In a case where the electrode film and the lid member are bonded via the adhesive layer, a rubber-based, acrylic-based, silicone-based, polyvinyl-based, polyester-based or polyurethane-based one is suitably used in view of safety and elimination of disadvantages involved at the time of moistening.

To bond the electrode film and the resin lid member via the adhesive layer, the resin lid member may be sealed with the blister container and then bonded to the adhesive layer formed on the electrode film, or the electrode film and the resin lid member may be laminated in advance before the blister container is sealed. It is desirable to laminate the electrode film and the resin lid member in advance, because a gap between the resin lid member and the adhesive layer is eliminated, and a loss of the dissolution liquid due to capillary action can be prevented effectively.

At the time of using the iontophoresis device configured as described above, the device is applied to the skin with the adhesive 8 provided on the top surface of the expanded sheet 4, the dissolution liquid-storing blister container 5 is pushed by fingers to break the resin lid member 7 by the projected portion 5b formed in the blister container to migrate the dissolution liquid 6 to the drug-loaded member 3 through the dissolution liquid passing portion 9 to mix with the drug. And, a current is passed to the electrode 2 to ionize the drug solution to introduce it into the body through the skin.

Embodiments of a method for producing an iontophoresis device of the present invention are described below.

### (Embodiment 1)

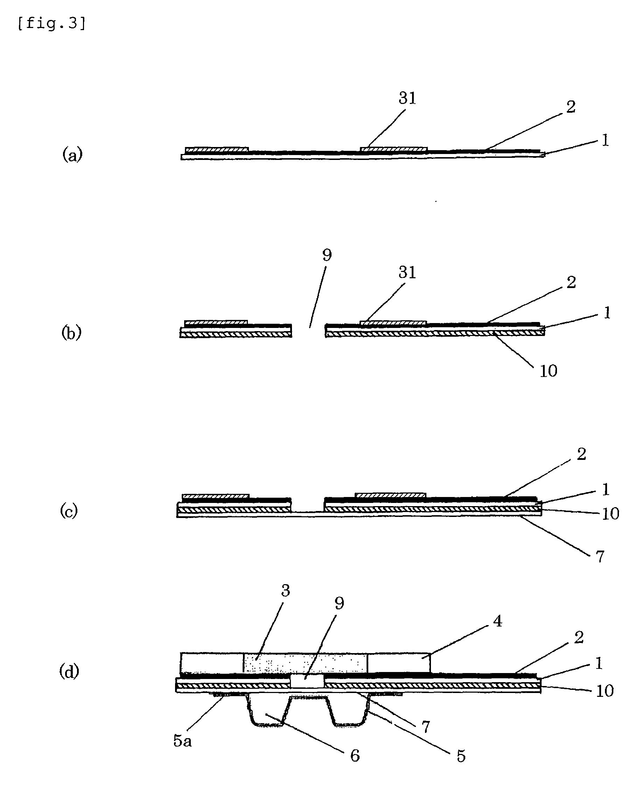

This embodiment relates to a method for producing the iontophoresis device configured as exemplified in Fig. 1. Fig. 3 shows a diagram of its production process.

The production method of this embodiment has a step of laminating a thermoplastic resin layer on the entire surface of a side of an electrode film to which a dissolution liquid storing container is bonded, a step of forming a dissolution liquid passing hole in the electrode film, a step of bonding a resin lid member to the thermoplastic resin layer by a heat lamination method, and a step of bonding the dissolution liquid storing container to the resin lid member.

First, the electrode layer 2 is formed on the base 1 such as a PET film, and a sealing layer 31 is formed on a prescribed region such as the peripheral portion of the electrode layer (Fig. 3(a)).

Then, the electrode film is obtained by laminating a thermoplastic resin layer 10 on the entire surface of the non-electrode-layer-forming side of the base 1 and forming a hole which becomes the dissolution liquid passing portion 9 by punching fabrication (Fig. 3(b)).

As the laminating method of the thermoplastic resin layer, various fabrication methods such as an explosion lamination method, a dry lamination method, a heat lamination method and the like can be used. The explosion lamination method extrudes the thermoplastic resin by a T-die to laminate, and the dry and heat lamination method forms a film by inflation or a T-die method and laminates it.

Then, the resin lid member 7 is bonded to the thermoplastic resin layer 10 by the heat lamination method to obtain a lid member-laminated electrode film (Fig. 3(c)).

The drug-loaded member 3 and the expanded sheet 4 are positioned on the sealing layer 31 formed on the electrode side of the lid member-laminated electrode film, and they were bonded by heat sealing. Then, the flange surface 5a of the dissolution liquid-storing blister container 5 into which the dissolution liquid 6 is charged is bonded to the resin lid member surface of the lid member-laminated electrode film to complete the iontophoresis device of the present invention (Fig. 3(d)). As this bonding method, various types of methods such as heat sealing, impulse sealing, ultrasonic sealing, high-frequency sealing and the like can be used.

In this embodiment, since the dissolution liquid passing hole 9 is formed after the thermoplastic resin layer 10 is formed on the electrode film, there is no thermoplastic resin layer 10 at the region of the dissolution liquid passing hole 9. Therefore, when the resin lid member 7 is broken at the time of use, the thermoplastic resin layer 10 does not affect on piercing strength, and the dissolution liquid and the drug can be mixed easily.

### (Embodiment 2)

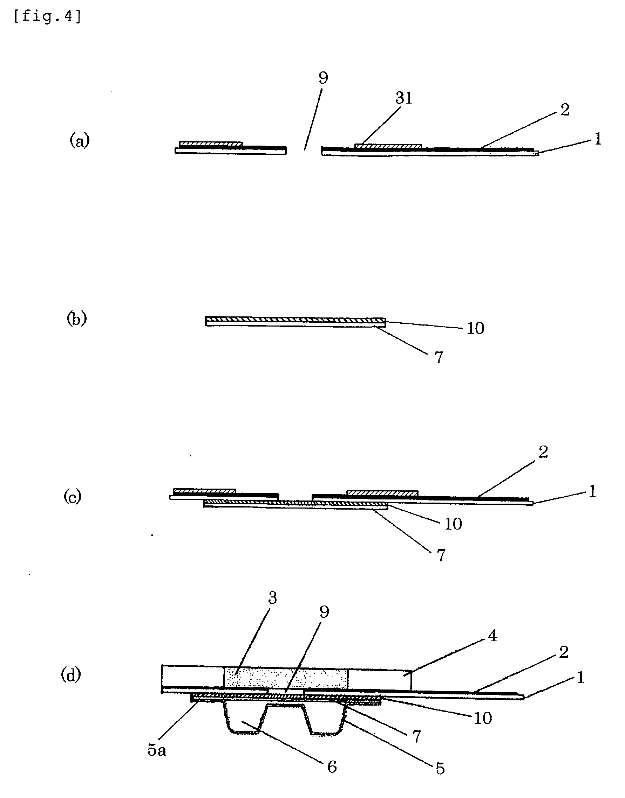

The thermoplastic resin layer was laminated on the electrode film in Embodiment 1, but Embodiment 2 is an example of laminating the thermoplastic resin layer on the resin lid member. Fig. 4 shows a diagram of its production process.

The production method of this embodiment has a step of forming a dissolution liquid passing hole in the electrode film, a step of laminating a thermoplastic resin layer on the resin lid member, a step of bonding the resin lid member to the electrode film via the thermoplastic resin layer according to the heat lamination method to cover the dissolution liquid passing hole, and a step of bonding the dissolution liquid storing container to the resin lid member.

First, an electrode layer 2 is formed on a base 1 of a PET film or the like, a sealing layer 31 is formed on a prescribed region such as a peripheral portion of the electrode layer, and a hole which becomes a dissolution liquid passing portion 9 is formed by punching fabrication (Fig. 4(a)).

And, a thermoplastic resin layer 10 is laminated on a resin lid member 7 (Fig. 4(b)).

Then, the resin lid member 7 is bonded to an electrode film via a thermoplastic resin layer 10 to cover the dissolution liquid passing portion 9 according to the heat lamination method to obtain a lid member-laminated electrode film (Fig. 4(c)).

A drug-loaded member 3 and an expanded sheet 4 were positioned on the sealing layer 31 which is formed on the electrode side of the lid member-laminated electrode film and bonded by heat sealing. Then, a flange surface 5a of a dissolution liquid-storing blister container 5 into which a dissolution liquid 6 is charged is bonded to a resin lid member surface of a lid member-laminated electrode film to complete an iontophoresis device of the present invention (Fig. 4(d)).

In a case where the thermoplastic resin layer is laminated on the resin lid member as in this embodiment, and especially when a thermoplastic resin which considerably affects on piercing strength is used, the region of the dissolution liquid passing hole 9 is determined to have a single layer structure of a resin lid member only, so that it is desirable to form a thermoplastic resin layer by using a resin film having an opening corresponding to the dissolution liquid passing hole.

### (Embodiment 3)

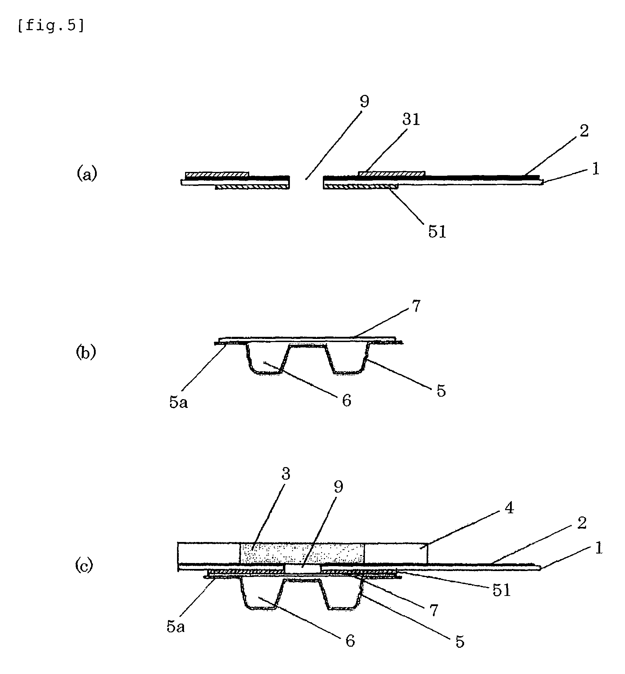

The resin lid member was bonded to the electrode film via the thermoplastic resin layer in Embodiments 1, 2. Embodiment 3 is an example of bonding a resin lid member to an electrode film via an adhesive layer, and Fig. 5 shows a diagram of its production process.

The production method of this embodiment has a step of forming an adhesive layer on only a part of an electrode film to which a dissolution liquid storing container is bonded, a step of forming a dissolution liquid passing hole in an inside region of the electrode film to which the adhesive layer was formed, a step of bonding a resin lid member to a flange of the dissolution liquid storing container, and a step of bonding the resin lid member bonded to the dissolution liquid storing container to the electrode film via the adhesive layer to cover the dissolution liquid passing hole.

First, an electrode layer 2 is formed on a base 1 of a PET film or the like, a sealing layer 31 is formed on a prescribed region such as a peripheral portion of the electrode layer, an adhesive layer 51 is coated on only a bonded portion of a non-electrode-layer-forming side of the base 1 with a dissolution liquid-storing blister container, and a hole which becomes a dissolution liquid passing portion 9 is formed by punching fabrication to obtain an electrode film (Fig. 5(a)).

As the adhesive coating method described above, there can be used various coating methods such as gravure coating, reverse coating, lip coating, die coating, comma coating, knife coating, screen printing, calender coating, hot melt-coating and the like.

And, a resin lid member 7 is bonded to a flange 5a of a dissolution liquid-storing blister container 5 in which the dissolution liquid 6 is charged (Fig. 5(b)).

Then, the drug-loaded member 3 and the expanded sheet 4 are provided on the sealing layer 31 which is formed on the electrode side of the electrode film and bonded by heat sealing. Then, the blister container 5 to which the resin lid member 7 was adhered was bonded to the adhesive layer-formed side of the electrode film to complete the iontophoresis device (Fig. 5(c)).

Since the dissolution liquid passing hole 9 is formed after the adhesive layer is formed on the electrode film in this embodiment, the region of the dissolution liquid passing hole 9 is free from the adhesive layer 51. Therefore, when the resin lid member 7 is broken at the time of use, the adhesive layer 51 does not affect on piercing strength, and the dissolution liquid and the drug can be mixed easily.

### (Embodiment 4)

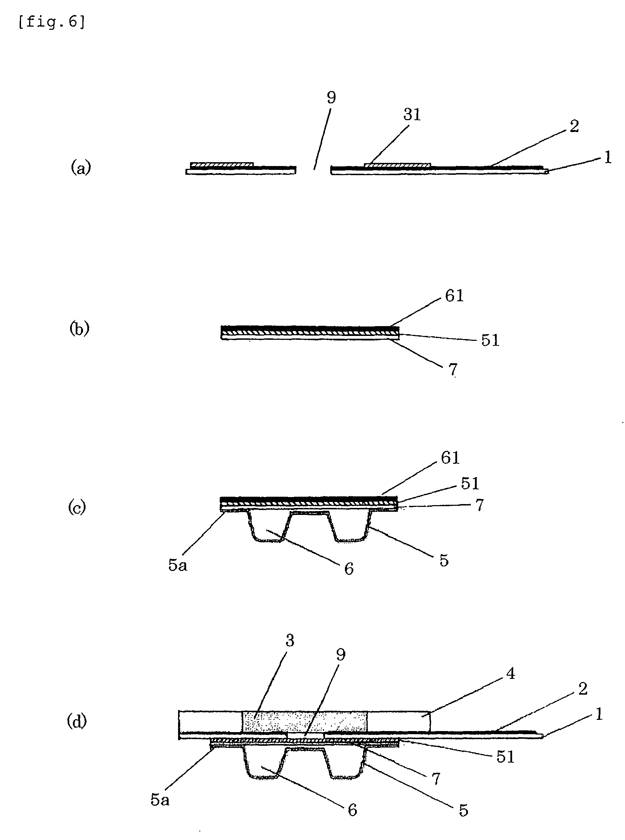

The adhesive layer was formed on the electrode film in Embodiment 3. Embodiment 4 is an example of forming the adhesive layer on the resin lid member, and Fig. 6 shows a diagram of its production process.

The production method of this embodiment has a step of forming a dissolution liquid passing hole in an electrode film, a step of bonding a non-adhesive-layer-forming side of a resin lid member having an adhesive layer formed on one side surface to a flange of a dissolution liquid storing container, and a step of bonding the resin lid member bonded to the dissolution liquid storing container to the electrode film via the adhesive layer to cover the dissolution liquid passing hole.

First, an electrode layer 2 is formed on a base 1 of a PET film or the like, a sealing layer 31 is formed on a prescribed region such as a peripheral portion of the electrode layer, and a hole which becomes a dissolution liquid passing portion 9 is formed by punching fabrication to obtain an electrode film (Fig. 6(a)).

An adhesive layer 51 is formed on a release surface of a release film 61, and a resin lid member 7 is laminated on the adhesive layer-formed side to obtain an adhesive layer-formed resin lid member (Fig. 6(b)).

Then, a non-adhesive-layer-forming side of the above-described adhesive layer-formed resin lid member is bonded to a flange 5a of a dissolution liquid-storing blister container 5 in which a dissolution liquid 6 is charged (Fig. 6(c)).

Then, a drug-loaded member 3 and an expanded sheet 4 are provided on the sealing layer 31 formed on the electrode side of the electrode film and bonded by heat sealing. The release film 61 of the adhesive layer-formed resin lid member applied to the blister container 5 is removed to bond to the electrode film via the adhesive layer 51 to cover the dissolution liquid passing hole 9 with the resin lid member 7 to complete the iontophoresis device (Fig. 6(d)).

In a case where the adhesive layer is coated on the lid member side as in this embodiment, and especially when an adhesive which considerably affects on piercing strength is used, the region of the dissolution liquid passing hole 9 is determined to have a single layer structure of a lid member only, so that it is desirable to coat the adhesive by partial coating.

As described above, the iontophoresis device of the present invention has the resin lid member, which is used as the lid member of the blister container, bonded to the electrode film to integrate the dissolution liquid-storing blister container with the electrode film, and since the insulating resin lid member is used as the lid member, the iontophoresis device realized has a structure that the dissolution liquid and the drug can be mixed by a simple operation, and it is free from a possibility of causing a leakage of electricity when current is applied and excellent in reliability and safety.

According to the method for producing the iontophoresis device of the present invention, the electrode film (or electrode film with a resin lid member) can be mass produced by using a material such as a rolled sheet, and the iontophoresis device excelling in reliability and safety can be mass produced easily.

### EXAMPLES

Specific examples of the present invention are described below but the present invention is not limited to them.

### [Example 1]

Example 1 is an example of producing an iontophoresis device by the production process shown in Fig. 3.

### (Formation of lid member-laminated electrode film)

Conductive carbon and a conductive silver chloride paste ink were screen printed on a base 1 of a biaxially stretched polyethylene terephthalate (PET) film having a thickness of 75 µm to form an electrode layer 2 having a thickness of 30 µm. Then, a sealing layer 31 having a thickness of 10 µm was formed by screen printing an ink composed of a thermoplastic saturated copolymer polyester resin on a prescribed region such as a peripheral portion of the electrode layer 2 (Fig. 3(a)).

Then, an anchor coating agent (trade name "TAKELAC" produced by Mitsuitakeda-Chem. Co.) was coated on the entire surface of a non-printed surface of the base, low-density polyethylene (trade name "Suntec LD" produced by ASAHIKASEI CORPORATION) was additionally laminated by the extrusion lamination method to form a thermoplastic resin layer 10, and a hole which became a dissolution liquid passing portion 9 was formed by punching fabrication (Fig. 3(b)).

Then, the thermoplastic resin layer 10 and a resin lid member 7 (trade name "PP-MONO-PTP" produced by FUJIMORI KOGYO CO., LTD.) were laminated by fusion bonding by the heat lamination method to obtain a lid member-laminated electrode film (Fig. 3(c)).

### (Formation of dissolution liquid-storing blister container)

A dissolution liquid-storing blister container 5 having a projection at the center portion was obtained by vacuum molding of a resin sheet for PTP (trade name "PP-MONO-PTP" produced by FUJIMORI KOGYO CO., LTD.).

### (Assembling of device)

An expanded olefin sheet 4 (trade name "Volara" produced by SEKISUI CHEMICAL CO., LTD.) and a drug-loaded nonwoven fabric 3 were positioned on the sealing layer 31 which was formed on the electrode side of the lid member-laminated electrode film and bonded mutually by heat sealing. Subsequently, a dissolution liquid 6 was charged into the dissolution liquid-storing blister container 5, the dissolution liquid passing portion 9 of a lid member-laminated electrode film was positioned at the center of the blister container, and the flange surface of the blister container was bonded to the lid member surface of the lid member-laminated electrode film by impulse sealing to complete the iontophoresis device.

Since the insulating resin lid member is used as the lid member for the iontophoresis device of this embodiment, the iontophoresis device obtained is free from a possibility of causing a leakage of electricity when current is applied different from a conventional aluminum lid member and excellent in reliability and safety. And, the thermoplastic resin layer is laminated on the entire surface of one side of the electrode film, and the resin lid member is heat laminated on the entire surface of the thermoplastic resin layer, so that a lid member-laminated electrode film can be mass-produced extremely efficiently by using a rolled member.

### [Example 2]

Example 2 is an example of producing an iontophoresis device by the production process shown in Fig. 3.

### (Formation of lid member-laminated electrode film)

Conductive carbon and a conductive silver chloride paste ink were screen printed on a base 1 of a biaxially stretched polyethylene terephthalate (PET) film having a thickness of 75 µm to form an electrode layer 2 having a thickness of 30 µm. Then, a sealing layer 31 having a thickness of 10 µm was formed by screen printing an ink composed of a thermoplastic saturated copolymer polyester resin on a prescribed region such as a peripheral portion of the electrode layer 2 (Fig. 3(a)).

Then, an adhesive (trade name "TAKELAC" produced by Mitsuitakeda-Chem. Co.) was coated on the entire surface of a non-printed surface of the base 1, a low-density polyethylene film (trade name "SUZULON L" produced by AICELLO CHEMICAL CO., LTD.) was additionally laminated by the dry lamination method to form a thermoplastic resin layer 10, and a hole which became a dissolution liquid passing portion 9 was formed by punching fabrication (Fig. 3(b)).

Then, the thermoplastic resin layer 10 and the resin lid member 7 were laminated by fusion bonding by the heat lamination method to obtain a lid member-laminated electrode film (Fig. 3(c)).

An iontophoresis device was completed by the same manner as in Example 1.

Since the insulating resin lid member is used as the lid member for the iontophoresis device of Example 2, the iontophoresis device obtained is free from a possibility of causing a leakage of electricity when current is applied different from a conventional aluminum lid member and excellent in reliability and safety. And, the thermoplastic resin layer is laminated on the entire surface of one side of the electrode film, and the resin lid member is heat laminated on the entire surface of the thermoplastic resin layer, so that a lid member-laminated electrode film can be mass-produced extremely efficiently by using a rolled member.

### [Example 3]

In Example 3, an iontophoresis device was produced in the same manner as in Example 1 except that a procedure of producing the lid member-laminated electrode film was different.

### (Formation of lid member-laminated electrode film)

An anchor coating agent (trade name "TAKELAC" produced by Mitsuitakeda-Chem. Co.) was coated on the entire surface of one side of a base 1 of a biaxially stretched polyethylene terephthalate (PET) film having a thickness of 75 µm, and low-density polyethylene (trade name "Suntec LD" produced by ASAHIKASEI CORPORATION) was additionally laminated by the extrusion lamination method to form a thermoplastic resin layer 10. Then, conductive carbon and a conductive silver chloride paste ink were screen printed on the PET surface of the base to form an electrode layer 2 having a thickness of 30 µm. Then, a sealing layer 31 having a thickness of 10 µm was formed by screen printing on a prescribed region such as a peripheral portion of the electrode layer 2 by using an ink composed of the thermoplastic saturated copolymer polyester resin, and a hole which became a dissolution liquid passing portion 9 was formed by punching fabrication (Fig. 3(b)).

Then, the thermoplastic resin layer 10 and a resin lid member 7 were laminated by fusion bonding by the heat lamination method to obtain a lid member-laminated electrode film (Fig. 3(c)).

An iontophoresis device was completed by the same manner as in Example 1.

Since the insulating resin lid member is used as the lid member for the iontophoresis device of Example 3, the iontophoresis device obtained is free from a possibility of causing a leakage of electricity when current is applied different from a conventional aluminum lid member and excellent in reliability and safety. And, the thermoplastic resin layer is laminated on the entire surface of one side of the electrode film, and the resin lid member is heat laminated on the entire surface of the thermoplastic resin layer, so that a lid member-laminated electrode film can be mass-produced extremely efficiently by using a rolled member.

### [Example 4]

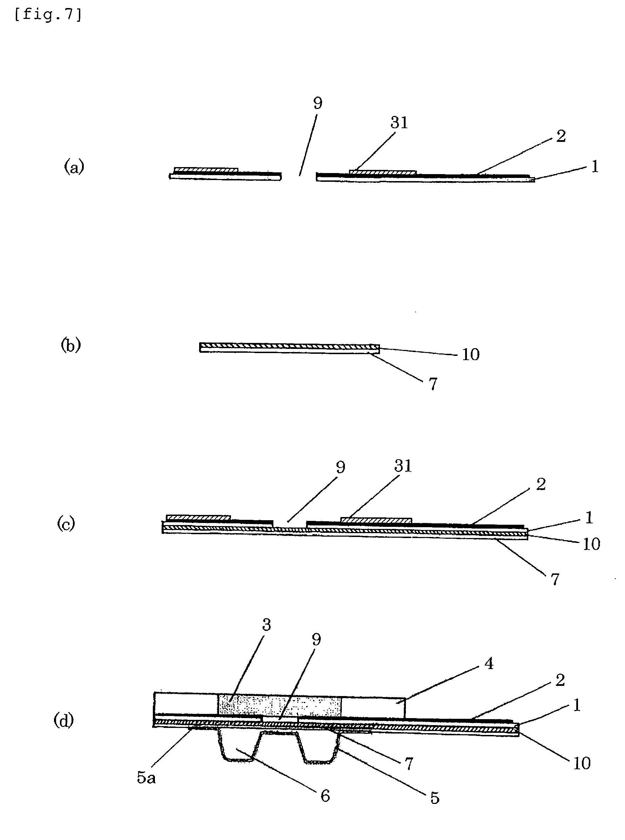

Example 4 is an example of producing an iontophoresis device by the production process shown in Fig. 7.

### (Formation of lid member-laminated electrode film)

Conductive carbon and a conductive silver chloride paste ink were screen printed on a base 1 of a biaxially stretched polyethylene terephthalate (PET) film having a thickness of 75 µm to form an electrode layer 2 having a thickness of 30 µm. Then, a sealing layer 31 having a thickness of 10 µm was formed by screen printing an ink composed of a thermoplastic saturated copolymer polyester resin on a prescribed region such as a peripheral portion of the electrode layer 2, and a hole which became a dissolution liquid passing portion 9 was formed by punching fabrication to obtain an electrode film (Fig. 7(a)).

Then, an adhesive resin (thermoplastic resin layer) film 10a (trade name "Melthene" produced by TOSOH CORPORATION) was sandwiched between a non-printed surface of the electrode film and a resin lid member 7, and they were laminated by fusion bonding by the heat lamination method to form a lid member-laminated electrode film.

Similar to Example 1, the drug-loaded portion and the dissolution liquid-storing blister container were bonded to the lid member-laminated electrode film to complete the iontophoresis device.

Since the insulating resin lid member is used as the lid member for the iontophoresis device of Example 4, the iontophoresis device obtained is free from a possibility of causing a leakage of electricity when current is applied different from a conventional aluminum lid member and excellent in reliability and safety. And, the resin lid member is heat laminated on the entire surface of one side of the electrode film via the thermoplastic resin layer (adhesive resin film 10a), so that a lid member-laminated electrode film can be mass-produced extremely efficiently by using a rolled member.

### [Example 5]

Example 5 is an example of producing an iontophoresis device by the production process shown in Fig. 4.

### (Formation of lid member-laminated electrode film)

Conductive carbon and a conductive silver chloride paste ink were screen printed on a base 1 of a biaxially stretched polyethylene terephthalate (PET) film having a thickness of 75 µm to form an electrode layer 2 having a thickness of 30 µm. Then, a sealing layer 31 having a thickness of 10 µm was formed by screen printing an ink composed of a thermoplastic saturated copolymer polyester resin on a prescribed region such as a peripheral portion of the electrode layer 2, and a hole which became a dissolution liquid passing portion 9 was formed by punching fabrication to obtain an electrode film (Fig. 4(a)).

Then, a thermoplastic resin 10 (trade name "Melthene" produced by TOSOH CORPORATION) and a resin lid member 7 were laminated by the extrusion lamination method (Fig. 4 (b)), and the thermoplastic resin layer 10 and the non-printed surface of the electrode film were laminated by fusion bonding by the heat lamination method to form a lid member-laminated electrode film (Fig. 4(c)).

Similar to Example 1, the drug-loaded portion and the dissolution liquid-storing blister container were fitted to the lid member-laminated electrode film to complete the iontophoresis device.

Since the insulating resin lid member is used as the lid member for the iontophoresis device of Example 5, the iontophoresis device obtained is free from a possibility of causing a leakage of electricity when current is applied different from a conventional aluminum lid member and excellent in reliability and safety.

### [Example 6]

Example 6 is an example of producing an iontophoresis device by the production process shown in Fig. 5.

### (Formation of electrode film)

Conductive carbon and a conductive silver chloride paste ink were screen printed on a base 1 of a biaxially stretched polyethylene terephthalate (PET) film having a thickness of 75 µm to form an electrode layer 2 having a thickness of 30 µm. Then, a sealing layer 31 having a thickness of 10 µm was formed by screen printing an ink composed of a thermoplastic saturated copolymer polyester resin on a prescribed region such as a peripheral portion of the electrode layer 2. Then, an adhesive layer 51 was formed on the non-printed surface of the base by partial coating an acrylic-based adhesive (trade name "SK-Dyne" produced by Soken Chemical & Engineering Co., Ltd.) on the portion bonded to the blister, and a hole which became a dissolution liquid passing portion 9 was formed by punching fabrication to obtain an electrode film (Fig. 5(a)).

### (Formation of dissolution liquid-storing blister container)

A dissolution liquid-storing blister container 5 having a projection at the center portion was obtained by vacuum molding of a resin sheet for PTP (trade name "PP-MONO-PTP" produced by FUJIMORI KOGYO CO., LTD.). A dissolution liquid 6 was charged into the dissolution liquid-storing blister container 5, and a resin lid member 7 (trade name "PP-MONO-PTP" produced by FUJIMORI KOGYO CO. , LTD.) was laminated on a flange of the blister container and sealed by heat sealing (Fig. 5(b)).

### (Assembling of device)

An expanded olefin sheet 4 (trade name "Volara" produced by SEKISUI CHEMICAL CO., LTD.) and a drug-loaded nonwoven fabric 3 were arranged on a sealing layer 31 formed on the electrode side of the electrode film and bonded by heat sealing. Subsequently, the blister container 5 to which the resin lid member 7 was applied was bonded to the adhesive layer-formed side of the electrode film to complete an iontophoresis device.

Since the insulating resin lid member is used as the lid member for the iontophoresis device of Example 6, the iontophoresis device obtained is free from a possibility of causing a leakage of electricity when current is applied different from a conventional aluminum lid member and excellent in reliability and safety.

### [Example 7]

Example 7 is an example of producing an iontophoresis device by the production process shown in Fig. 6.

### (Formation of electrode film)

Conductive carbon and a conductive silver chloride paste ink were screen printed on a base 1 of a biaxially stretched polyethylene terephthalate (PET) film having a thickness of 75 µm to form an electrode layer 2 having a thickness of 30 µm. Then, a sealing layer 31 having a thickness of 10 µm was formed by screen printing an ink composed of a thermoplastic saturated copolymer polyester resin on a prescribed region such as a peripheral portion of the electrode layer 2. Then, a hole which became a dissolution liquid passing portion 9 was formed by punching fabrication to obtain an electrode film (Fig. 6(a)).

### (Formation of adhesive layer-formed resin lid member)

An adhesive layer 51 was formed on a release surface of a release film 61 by comma coating an acrylic-based adhesive (trade name "SK-Dyne" produced by Soken Chemical & Engineering Co., Ltd.), and a resin lid member 7 (trade name "PP-MONO-PTP" produced by FUJIMORI KOGYO CO., LTD.) was laminated on the adhesive layer-formed side (Fig. 6(b)).

### (Formation of dissolution liquid-storing blister container)

A dissolution liquid-storing blister container 5 having a projection at the center portion was obtained by vacuum molding of a resin sheet for PTP (trade name "PP-MONO-PTP" produced by FUJIMORI KOGYO CO., LTD.). A dissolution liquid 6 was charged into the dissolution liquid-storing blister container 5, the adhesive layer-formed resin lid member was laminated on a flange of the blister container and sealed by heat sealing (Fig. 6(c)).

### (Assembling of device)

An expanded olefin sheet 4 (trade name "Volara" produced by SEKISUI CHEMICAL CO., LTD.) and a drug-loaded nonwoven fabric 3 were arranged on a sealing layer 31 formed on an electrode side of an electrode film, and bonded by heat sealing. Subsequently, a release film 61 of an adhesive layer-formed resin lid member applied to the blister container was removed to bond to the electrode film so as to complete the iontophoresis device.

Since the insulating resin lid member is used as the lid member for the iontophoresis device of Example 7, the iontophoresis device obtained is free from a possibility of causing a leakage of electricity when current is applied different from a conventional aluminum lid member and excellent in reliability and safety.

### BRIEF DESCRIPTION OF THE DRAWINGS

[Fig. 1] Diagram showing an embodiment of an iontophoresis device according to the present invention, where (a) is a sectional view, and (b) is a perspective view.
[Fig. 2] Diagram showing a dissolution liquid-storing blister container according to an embodiment of an iontophoresis device according to the present invention, where (a) is a top view, and (b) is a A-A' sectional view of (a).
[Fig. 3] Diagram showing a production process for description of an example of a method for producing an iontophoresis device of the present invention.
[Fig. 4] Diagram showing a production process for description of another example of a method for producing an iontophoresis device of the present invention.
[Fig. 5] Diagram showing a production process for description of an another example of a method for producing an iontophoresis device of the present invention.
[Fig. 6] Diagram showing a production process for description of another example of a method for producing an iontophoresis device of the present invention.
[Fig. 7] Diagram showing a production process for description of another example of a method for producing an iontophoresis device of the present invention.

### EXPLANATION OF REFERENCE NUMERALS

1: Base of electrode film
2: Electrode layer
3: Drug-loaded member
4: Expanded sheet
5: Dissolution liquid storing container (dissolution liquid-storing blister container)
5a: Flange
5b: Projected portion (projection)
6: Dissolution liquid
7: Resin lid member
8: Adhesive
9: Dissolution liquid passing portion
10: Thermoplastic resin layer
31: Sealing layer
51: Adhesive layer
61: Release film

## Claims

1. An iontophoresis device, comprising at least an electrode film having an electrode layer formed on a base, a drug-loaded member arranged on the electrode-layer-forming side of the electrode film, and a dissolution liquid storing container arranged on the non-electrode-layer-forming side of the electrode film, wherein:
the electrode film is provided with a dissolution liquid passing hole, a resin lid member is bonded to the electrode film via a thermoplastic resin layer or an adhesive layer to cover the dissolution liquid passing hole, and a flange of the dissolution liquid storing container is bonded to the electrode film via the resin lid member.

2. The iontophoresis device according to claim 1, wherein the surface of the resin lid member positioned at the dissolution liquid passing hole does not have the thermoplastic resin layer or the adhesive layer.

3. A method for producing an iontophoresis device which comprises at least an electrode film having an electrode layer formed on a base, a drug-loaded member arranged on the electrode-layer-forming side of the electrode film, and a dissolution liquid storing container arranged on the non-electrode-layer-forming side of the electrode film, comprising:
laminating a thermoplastic resin layer on the entire surface on the side of the electrode film to which the dissolution liquid storing container is bonded,
forming a dissolution liquid passing hole in the electrode film,
bonding a resin lid member to the thermoplastic resin layer by a heat lamination method, and
bonding the dissolution liquid storing container to the resin lid member.

4. A method for producing an iontophoresis device which comprises at least an electrode film having an electrode layer formed on a base, a drug-loaded member arranged on the electrode-layer-forming side of the electrode film, and a dissolution liquid storing container arranged on the non-electrode-layer-forming side of the electrode film, comprising:
forming a dissolution liquid passing hole in the electrode film,
laminating a thermoplastic resin layer on a resin lid member,
bonding the resin lid member to the electrode film via the thermoplastic resin layer to cover the dissolution liquid passing hole by a heat lamination method, and
bonding the dissolution liquid storing container to the resin lid member.

5. A method for producing an iontophoresis device which comprises at least an electrode film having an electrode layer formed on a base, a drug-loaded member arranged on the electrode-layer-forming side of the electrode film, and a dissolution liquid storing container arranged on the non-electrode-layer-forming side of the electrode film, comprising:
forming an adhesive layer on only a part of the electrode film to which the dissolution liquid storing container is bonded,
forming a dissolution liquid passing hole in a region of the inner side of the electrode film where the adhesive layer is formed,
bonding a resin lid member to a flange of the dissolution liquid storing container, and
bonding the resin lid member bonded to the dissolution liquid storing container to the electrode film via the adhesive layer to cover the dissolution liquid passing hole.

6. A method for producing an iontophoresis device which comprises at least an electrode film having an electrode layer formed on a base, a drug-loaded member arranged on the electrode-layer-forming side of the electrode film, and a dissolution liquid storing container arranged on the non-electrode-layer-forming side of the electrode film, comprising:
forming a dissolution liquid passing hole in the electrode film,
bonding the non-adhesive-layer-forming side of a resin lid member which has an adhesive layer formed on its one surface to a flange of the dissolution liquid storing container, and
bonding the resin lid member which is bonded to the dissolution liquid storing container to the electrode film via the adhesive layer to cover the dissolution liquid passing hole.
